# FASCICULE DE BREVET EUROPEEN

(11) **EP 1 341 468 B1**
(45) Date de publication et mention de la délivrance du brevet: **03.02.2010**
(21) Numéro de dépôt: 01992535.3
(22) Date de dépôt: 06.11.2001
(51) Int. Cl.: A61B 19/00, A61B 17/15

(54) **SYSTEME DE DETERMINATION DE LA POSITION D'UNE PROTHESE DU GENOU**
SYSTEM ZUR BESTIMMUNG DER POSITION EINER KNIEPROTHESE
SYSTEM FOR DETERMINING THE POSITION OF A KNEE PROSTHESIS

(30) Priorité: 06.11.2000 FR 0014173
(43) Date de publication de la demande: 10.09.2003
(73) Titulaire: Perception Raisonnement Action En Medecine, 38700 La Tronche (FR)
(72) Inventeur: LAVALLEE, Stéphane, F-38410 Saint Martin d'Uriace (FR)
(74) Mandataire: de Beaumont, Michel
(86) Numéro de dépôt international: PCT/FR2001/003423
(87) Numéro de publication internationale: WO 2002/036031

(56) Documents cités:
- EP-A- 0 920 838
- WO-A-98/40037
- WO-A-99/23956
- WO-A-99/60939
- FR-A- 2 775 889
- US-A- 5 564 437
- US-A- 5 722 978
- KIENZLE T C ET AL: "TOTAL KNEE REPLACEMENT" IEEE ENGINEERING IN MEDICINE AND BIOLOGY MAGAZINE,IEEE INC. NEW YORK,US, vol. 14, no. 3, 1 mai 1995 (1995-05-01), pages 301-306, XP000505086 ISSN: 0739-5175

## Description

La présente invention concerne un système de détermination de la position d'une prothèse permettant d'assister un chirurgien à poser des prothèses de genou, à l'aide de l'informatique présente au bloc opératoire.

L'objectif général de la chirurgie du genou assistée par ordinateur est de déterminer une position optimale des prothèses sur le tibia, sur le fémur, et éventuellement sur la rotule selon des critères géométriques et dynamiques, et de proposer des dispositifs permettant de placer effectivement les prothèses à l'endroit optimum. On cherche idéalement à restaurer un alignement des centres de la hanche, du genou et de la cheville, pour un genou placé en extension, tout en équilibrant la position de la rotule et les tensions des ligaments au cours d'un mouvement de flexion et tout en ayant de bons ajustements des prothèses avec les os. Dans certains cas, on ne remplace qu'une partie du genou, par exemple un des deux condyles du fémur mais les critères restent identiques.

Les méthodes classiques utilisent des ancillaires mécaniques réglables et ajustables en fonction des données radiologiques de chaque patient. Ces méthodes sont imprécises et ne permettent pas de réaliser une pose de prothèse idéale dans tous les cas, ce qui conduit parfois le chirurgien à ajuster progressivement les positions des prothèses, ce qui est long et difficile, ou à se contenter d'un résultat de qualité moyenne.

Pour remédier à ces inconvénients, des systèmes de chirurgie assistée par ordinateur utilisant des capteurs de position, des ordinateurs et éventuellement des robots ont été développés.

En général, dans les systèmes classiques de chirurgie assistée par ordinateur on fixe un élément repérable, connu aussi sous le nom de repère de mesure, à un os ou une structure anatomique et on suit ses déplacements à l'aide d'un capteur de position, connu aussi sous le nom de système de localisation ou encore système tridimensionnel de positionnement. Un tel capteur de position peut être un ensemble de caméras qui repèrent la position et l'orientation de repères de mesure constitués chacun d'au moins trois diodes infrarouges ou marqueurs réfléchissants. On peut aussi utiliser de nombreuses technologies acoustiques, mécaniques ou magnétiques permettant de repérer la position et l'orientation relatives de deux repères de position fixés sur des structures anatomiques, des instruments chirurgicaux, des palpeurs de numérisation ou des capteurs divers comme cela est décrit dans le chapitre 1 du livre "Computer Integrated Surgery", MIT Press, 1996, R. Taylor ed., intitulé "Multimodal Information for Computer-Integrated Surgery", pages 5-21, de R. Moesges et S. Lavallée. L'un des repères de position peut également faire office de capteur de position, comme c'est le cas dans les systèmes magnétiques de petite taille. A l'aide de ces capteurs de position, on peut utiliser un palpeur pour numériser des points sur la surface des structures équipées d'un repère de position, on peut aussi enregistrer des mouvements entre deux os équipés de repères de position.

La plupart des systèmes de chirurgie assistée par ordinateur existant pour assister la pose d'une prothèse totale du genou nécessitent l'utilisation d'images médicales acquises avant une intervention par des moyens puissants tels que le scanner tomodensitométrique (TDM) ou l'imagerie par résonance magnétique (IRM), comme cela est décrit dans les brevets de ORTHOSOFT Inc (WO 99/60939), Eric Brosseau et al., ou de Scott Delp et al. (US-A-5682886). Sur la base de ces images, un médecin peut planifier et simuler partiellement une intervention chirurgicale, puis des techniques complexes de recalage entre les images du patient et la position du patient sur table d'opération permettent de suivre et reproduire les stratégies planifiées. Cependant, l'acquisition de telles images en routine clinique est compliquée à gérer, elle a un coût non négligeable, des risques d'erreurs sont associés aux procédés de recalage, et l'utilisation d'un scanner génère une dose de rayons X non négligeable pour le patient. De plus, de tels systèmes n'utilisent pas les informations cinématiques et dynamiques que l'on peut acquérir facilement au début d'une opération, ils ne permettent donc pas d'obtenir directement et facilement la position idéale des prothèses.

Une alternative consiste à n'utiliser que des informations acquises juste avant le début de l'opération à l'aide de capteurs de position. De telles méthodes sont toutefois limitées dans leur usage car elles ne permettent que de collecter certaines informations très simples pour rester dans des limites de temps raisonnable.

Dans cette catégorie, les méthodes les plus largement utilisées se basent sur la recherche d'un alignement des trois centres de rotation du genou, de la hanche et du tibia, comme le décrivent par exemple les documents WO-A-95/00075 d'ANDRONIC et WO-A-98/40037 d'AESCULAP. Mais, dans ces méthodes, la détermination du centre du genou est rendue difficile et imprécise par les variations anatomiques fortes de chaque genou qui doit subir une intervention, la définition même du centre d'un genou pathologique étant sujet de débats et controverses.

La présente invention vise à utiliser un équipement simple comprenant un ordinateur, un écran, un système tridimensionnel de positionnement, un repère de position fixé sur chaque os de l'articulation et un repère de position placé sur un guide de perçage.

Un objet de l'invention est de proposer un système automatique de détermination de la meilleure taille, position et orientation pour chaque implant posé sur un ou plusieurs plans de coupe osseuse, tel qu'une prothèse classique de tibia et de fémur.

Un autre objet de l'invention est de proposer un tel système dans lequel la surface complète de chaque os est obtenue par déformation d'un modèle générique au bloc opératoire, sans images IRM ou TDM.

Un autre objet de l'invention est de proposer un tel système dans lequel le degré de précision en tout point de l'image de la surface de l'os est indiqué par une couleur.

Un autre objet de l'invention est de proposer un tel système dans lequel on tienne compte d'informations ligamentaires pour équilibrer les tensions entre chaque os de l'articulation du genou.

Un autre objet de l'invention est de proposer un tel système dans lequel on prévoit un guide de perçage particulièrement simple à mettre en place pour assurer ensuite le positionnement précis d'un guide de coupe.

Un avantage de l'invention est qu'elle permet de s'affranchir de la détermination d'un centre du genou sur le patient. Au lieu d'aligner un quelconque centre du genou pathologique avec le centre de la hanche et de la cheville, l'invention prévoit d'aligner le centre de la prothèse du genou pathologique avec le centre de la hanche et de la cheville.

Un autre avantage de l'invention est qu'elle permet de déterminer automatiquement une position théorique idéale de l'ensemble des implants devant être fixés sur chaque os en tenant compte de tous les critères essentiels et de laisser ensuite le chirurgien modifier les paramètres par rapport à la référence dite idéale, juste avant la réalisation des actes chirurgicaux permettant de placer les implants.

Un autre avantage de l'invention est qu'elle s'affranchit de tous les procédés d'acquisition et recalage d'images médicales TDM ou IRM.

Pour atteindre ces objets, la présente invention prévoit un système automatique selon la révendication 1 de détermination de la position théorique idéale d'une prothèse du genou comprenant, pour la détermination de la position d'une prothèse tibiale, des moyens adaptés :
déterminer la forme de la partie proximale du tibia et sa position par rapport au centre de l'articulation de la cheville ;
déterminer par l'utilisateur un point haut de référence du plateau tibial ;
calculer de manière itérative la position, l'orientation et la taille par rapport au tibia de la prothèse tibiale et du plan de coupe correspondant à la prothèse tibiale en tenant compte des contraintes suivantes :
   - la perpendiculaire au plan de coupe passant par le centre de la prothèse passe aussi par le centre de l'articulation de la cheville,
   - le plan de coupe est à une distance dudit point haut de référence égale à l'épaisseur de la prothèse à fixer,
   - le grand côté de la prothèse est centré sur le grand côté de la section du tibia dans le plan de coupe,
   - le bord antérieur du petit côté de la prothèse est à une distance prédéterminée du bord antérieur du petit côté de la section du tibia dans le plan de coupe ; et des moyens adaptés à
déterminer l'orientation dans le plan de coupe de la prothèse de façon que le grand côté de la prothèse soit parallèle à l'axe horizontal du genou.

Selon un mode de réalisation de la présente invention, ce système comprend en outre, pour la détermination de la position d'une prothèse fémorale, des moyens adaptés à :
déterminer la position relative de la partie distale du fémur par rapport au centre de l'articulation de la hanche ;
calculer la position, l'orientation et la taille par rapport au fémur de la prothèse fémorale et du plan de coupe correspondant à la prothèse fémorale en tenant compte des contraintes suivantes :
   - la perpendiculaire au plan de coupe passant par le centre de la prothèse passe aussi par le centre de l'articulation de la hanche,
   - le plan de coupe distal est à une distance du point le plus distal sur un des condyles égale à l'épaisseur de la prothèse à fixer,
   - le plan de coupe postérieure est à une distance du point le plus postérieur sur un des condyles égale à l'épaisseur de la prothèse à fixer,
   - le grand côté de la prothèse est centré sur le grand côté de la section du fémur dans le plan de coupe,
   - la taille maximale de la prothèse est telle que le bord de la prothèse est le plus proche possible mais à l'intérieur de la surface du fémur; et
déterminer l'orientation dans le plan de coupe de la prothèse de façon que le grand côté de la prothèse soit parallèle à l'axe horizontal du genou.

Selon un mode de réalisation de la présente invention, l'orientation et la position latérale de la prothèse fémorale sont ajustées par un alignement du plan de la trochlée de la prothèse fémorale avec la trajectoire du centre de la rotule numérisée au cours d'un mouvement de flexion de genou grâce à un repère de position placé sur la face externe de la rotule.

Selon un mode de réalisation de la présente invention, ce système comprend en outre, pour la détermination des formes et positions du tibia, du fémur, du centre de l'articulation de la cheville et du centre de l'articulation de la hanche :
des éléments repérables en position dans un système tridimensionnel de positionnement, fixables au moins au tibia et au fémur,
un palpeur, un dispositif échographique, et/ou un dispositif radiographique pour repérer dans ledit système tridimensionnel de positionnement la position de divers points du tibia et du fémur par rapport auxdits éléments repérables, et
des moyens pour déformer et ajuster un modèle préétabli des os de la jambe en utilisant les informations de position recueillies.

Selon un mode de réalisation de la présente invention, ledit modèle comprend également une modélisation des positions des attaches des ligaments de l'articulation du genou, cette modélisation est adaptée au patient en même temps que le modèle de la surface osseuse, et ledit modèle comprend également les valeurs des élongations maximales de chaque ligament, déterminées expérimentalement en faisant bouger la jambe du patient non encore opérée pour repérer les limites de déplacement liées aux ligaments existants.

Selon un mode de réalisation de la présente invention, ce système comprend en outre des moyens adaptés à :
simuler sur l'image de l'ensemble fémur-tibia la mise en position des prothèses liées entre elles pour différentes positions de flexion, et la position des ligaments du genou ;
en déduire les limites de mouvement qu'aura la jambe munie des prothèses avec les ligaments existants ; et
tenir compte de cette information pour modifier la position théorique idéale de la prothèse du genou, et/ou pour suggérer une intervention sur les ligaments.

Selon un mode de réalisation de la présente invention, ledit palpeur a une extrémité en forme de boule et les repérages par le palpeur sont effectués de façon dynamique tandis que l'on déplace ladite boule contre la partie à analyser, le système de positionnement tridimensionnel étant conçu pour déterminer les positions instantanées du centre de la boule et un système de reconstitution de surface à partir d'un modèle déformable étant conçu pour effectuer une correction correspondant au rayon de la boule.

Selon un mode de réalisation de la présente invention, on forme une image du modèle ajusté et on affiche cette image en affectant ses diverses zones de couleurs ou d'épaisseur caractérisant la densité de points relevée dans cette zone par le palpeur, ce qui indique le degré de précision du modèle de la zone correspondante.

Selon un mode de réalisation de la présente invention, ce système comprend des moyens adaptés à :
déterminer par le calcul des emplacements auxquels le tibia/fémur doit être percé pour mettre en place un guide de coupe,
pointer ces emplacements sur l'image affichée du tibia/fémur,
afficher en permanence sur l'image reconstituée du tibia/fémur, l'image d'un guide de perçage muni de moyens de repérage dans ledit système tridimensionnel de positionnement, ce guide comportant des tubes de même écartement que des tubes du guide de coupe destinés à être montés sur des mèches fixées dans l'os,
et le guide de perçage comprend un picot central pouvant être mis en appui sur l'os et dont l'image doit coïncider avec un point cible prépositionné sur l'image affichée du tibia/fémur.

Selon un mode de réalisation de la présente invention, le guide de perçage comprend en outre des moyens de calage, actionnables une fois que le guide a été amené en position.

Selon un mode de réalisation de la présente invention, les éléments repérables respectivement associés au tibia, au fémur et au palpeur sont géométriquement différentiés.

Selon un mode de réalisation de la présente invention, l'élément repérable associé au tibia a la forme de la lettre T, l'élément repérable associé au fémur a la forme de la lettre F, l'élément repérable associé au palpeur a la forme de la lettre P.

Selon un mode de réalisation de la présente invention, la position du composant rotulien de la prothèse est obtenue par des moyens adaptés à :
déterminer dans un système tridimensionnel de positionnement, la trajectoire d'un élément repérable en position fixé à la face externe de la rotule au cours d'un mouvement de flexion du genou,
déterminer le point de la face interne de la rotule qui coïncide le mieux avec le centre de la gorge de la prothèse du fémur pour une zone angulaire de flexion choisie,
guider le placement d'une mèche de perçage vers ledit point.

Ces objets, caractéristiques et avantages, ainsi que d'autres de la présente invention seront exposés en détail dans la description suivante de modes de réalisation particuliers faite à titre non-limitatif en relation avec les figures jointes parmi lesquelles :
les figures 1A et 1B représentent une vue de face et une vue de côté d'un ensemble fémur-tibia ;
les figures 2A et 2B illustrent un fémur et un palpeur associé ;
les figures 3A et 3B représentent une partie extrême distale d'un fémur et des zones colorisées sur celui-ci ;
la figure 4 représente un modèle d'assemblage fémur-tibia et de ligaments associés ;
les figures 5A à 5C illustrent le montage d'une prothèse selon l'invention ;
les figures 6A à 6C illustrent des étapes mises en oeuvre par le système selon la présente invention ;
la figure 7 représente un outil de perçage selon la présente invention ;
la figure 8 représente des cibles de détermination de position d'un outil de perçage selon la présente invention ;
la figure 9 représente une variante de l'outil de perçage selon la présente invention ;
la figure 10 représente une autre variante de l'outil de perçage selon la présente invention ; et
la figure 11 représente une autre variante de l'outil de perçage selon la présente invention.

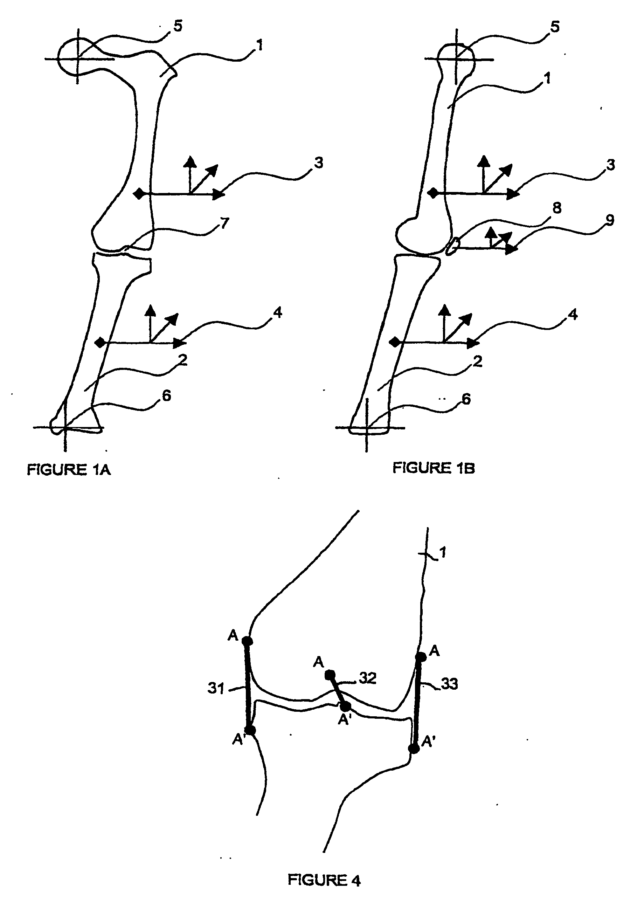

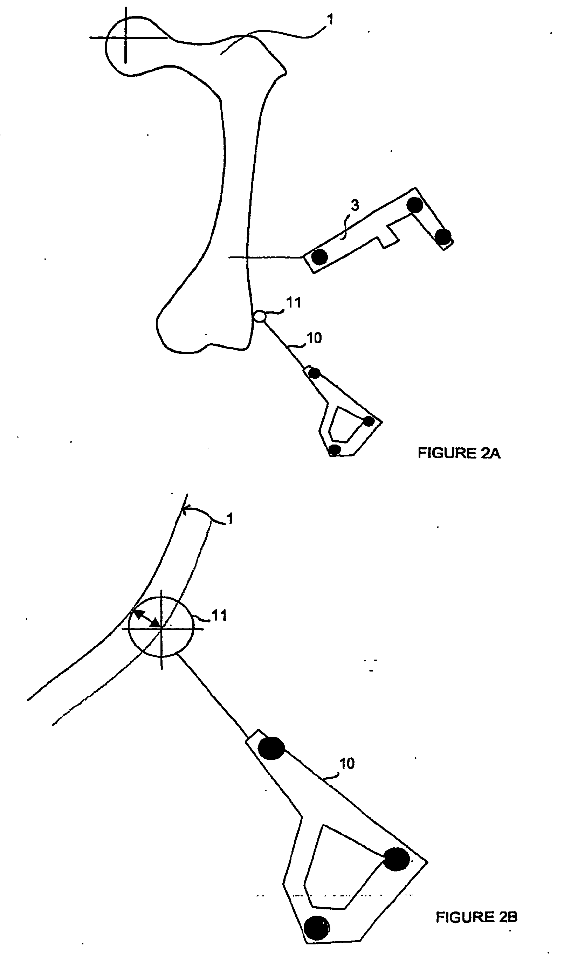

Pour effectuer une intervention selon la présente invention, on place les os dans le champ d'un système de positionnement tridimensionnel. Ensuite, comme l'illustrent la figure 1A en vue de côté et la figure 1B en vue de face, on analyse des éléments repérables par le système de positionnement tridimensionnel fixés respectivement sur le fémur 1 et sur le tibia 2 d'un patient, respectivement 3 et 4. Ces éléments repérables sont généralement des pointes vissées dans les os et dont une extrémité est munie de marqueurs qui peuvent être des disques réfléchissants, des sphères réfléchissantes ou des diodes infrarouges. Comme l'illustre la figure 2A, pour éviter tout risque d'erreur, l'invention prévoit que l'élément repérable 3 fixé sur le fémur a la forme de la lettre F, avec au moins trois marqueurs fixés sur les extrémités des branches et sur les coins du F. Ce dispositif permet d'avoir la plus grande lettre possible, ce qui évite toute confusion possible, avec des marqueurs le plus distant possible, ce qui augmente la précision du système, tout en formant une forme compacte. De la même façon, l'élément repérable fixé au tibia a la forme de la lettre T avec trois marqueurs fixés aux extrémités des branches du T. Tous les éléments repérables peuvent ainsi être associés à une lettre, P pour le palpeur, R pour la rotule, H pour l'humérus, etc. contenant des marqueurs sur les extrémités de ses branches et sur ses coins.

La référence 5 désigne le centre de rotation de la hanche entre le fémur et l'os iliaque, la référence 6 le centre de la cheville entre le tibia et le pied, et la référence 7 l'articulation du genou entre le fémur et le tibia. En figure 1B, on e désigné par la référence 8 la rotule et par la référence 9 un élément repérable fixé à la rotule sur sa face externe.

L'invention prévoit d'utiliser des moyens adaptés à déterminer la déformation d'un modèle générique pour aller épouser des points numérisés sur l'os réel. Le modèle est en général constitué d'un ensemble de plusieurs centaines de facettes triangulaires reliées entre elles sur leurs arêtes et sommets. On peut utiliser des méthodes décrites dans le chapitre 16 du livre "Brain Warping", Toga ed., Academic Press, 1999, intitulé "Elastic registration and Inference using Oct-Tree Splines", pages 282-296, S. Lavallée et al., qui consistent à déformer le moins possible un maillage volumique qui englobe et entraîne avec lui les points de surface numérisés jusqu'à les faire coïncider avec la surface du modèle, puis à inverser la fonction de transformation ainsi obtenue pour déformer le modèle générique vers les points numérisés. Dans une telle méthode, on commence par chercher une déformation globale qui amène certains points anatomiques particuliers palpés par l'utilisateur vers leurs homologues du modèle, puis on affine la déformation en diminuant progressivement la somme des carrés des distances entre tous les points palpés et la surface du modèle. Des méthodes de calculs de distance entre des points et une surface représentée par des facettes triangulaires sont largement connues dans la littérature de la géométrie tridimensionnelle informatisée. On obtient ainsi une fonction de déformation F transformant les coordonnées (X,Y,Z) d'un point exprimées dans le repère de position en des coordonnées (X',Y',Z') du point exprimées dans un repère associé au modèle. Pour obtenir le modèle déformé, on recherche le point antécédent (X,Y,Z) de chaque point du modèle (XM, YM, ZM) par la fonction F en minimisant itérativement l'écart entre (XM, YM, ZM) et F(X, Y, Z) et on conserve les liens géométriques appliqués entre les points du modèle. On peut aussi utiliser des méthodes décrites dans l'article "Building a complète surface model from sparse data using statistical shape models : application to computer assisted knee surgery" de M Fleute et S Lavallée, paru dans Medical Image Computing And Computer-Assisted Intervention - MICCAI'98, Spinger-Verlag LNCS Series, pages 880-887, octobre 1998. De telles méthodes sont robustes car elles utilisent des modèles statistiques. On réalise ainsi une interpolation intelligente des points permettant de construire des surfaces complexes à l'aide de peu de points, donc facilement et rapidement. On peut également combiner les deux approches citées en commençant par rechercher la déformation d'un modèle statistique selon la méthode décrite dans l'article de Fleute précédemment cité puis en continuant par une déformation d'un maillage volumique selon la méthode décrite dans l'article de S. Lavallée paru dans le livre "Brain Warping" précédemment cité.

Pour déterminer et numériser des points sur la surface des os, on utilise un palpeur repérable dans le système tridimensionnel de positionnement. De façon classique, on peut utiliser un palpeur à extrémité pointue pour obtenir des points sur la surface avec une bonne précision, mais un tel palpeur s'accroche sur l'os et il est difficile de numériser de nombreux points à la volée.

L'invention prévoit, comme l'illustrent les figures 2A et 2B d'utiliser un palpeur 10 dont l'extrémité en contact avec la zone à détecter comporte une portion sphérique dont le rayon peut être supérieur à 0, 5 mm (de préférence de 0, 5 mm à 3 mm) . A partir d'un instant où le chirurgien ou autre opérateur envoie un ordre par une pression sur une commande à pied ou à main, ou par commande vocale, on peut alors numériser de nombreux points à la volée en faisant glisser la partie sphérique sur la surface de l'os mais on enregistre des points décalés d'une valeur égale au rayon de la sphère à l'extérieur de la surface réelle. Ce décalage devra être compensé. Pour compenser le rayon de la sphère du palpeur, l'invention prévoit des moyens pour minimiser progressivement la somme des distances entre chaque point et la surface du modèle auxquelles on soustrait la valeur du rayon du palpeur pourvu que les distances à la surface soient positives à l'extérieur de la surface et de signe négatif à l'intérieur de la surface.

Malgré l'utilisation de la sphère, il serait fastidieux de demander à l'opérateur de numériser toutes les parties utiles de la surface de- l'os Cela peut demander un travail manuel considérable et les surfaces reconstruites peuvent être assez imprécises voire erronées.

Dans les cas où on ne peut pas accéder aux points de surface directement, on peut utiliser une sonde échographique équipée d'un repère de position pour repérer des points sur l'os à travers la peau, comme cela est décrit dans le chapitre 32 du livre "Computer Integrated Surgery", MIT Press, 1996, R. Taylor ed., intitulé "Computer-assisted spinal surgery using anatomy-based registration", pages 434-437, de S. Lavallée et al. On peut également construire les surfaces en utilisant quelques images radiographiques obtenues au bloc opératoire ou en salle de radiologie. Si on utilise des radios acquises hors du bloc opératoire, on doit alors recaler les modèles ainsi reconstruits sur les données opératoires. De telles techniques sont décrites dans l'article "Nonrigid 3D/2D registration of images using statistical models" de M. Fleute et S. Lavallée, paru dans Medical Image Computing And Computer-Assisted Intervention - MICCAI'99, Spinger-Verlag LNCS Séries 1679, pages 138-147, octobre 1999. Cet article décrit également comment on peut combiner des informations radiologiques et des informations de position pour construire des surfaces 3D.

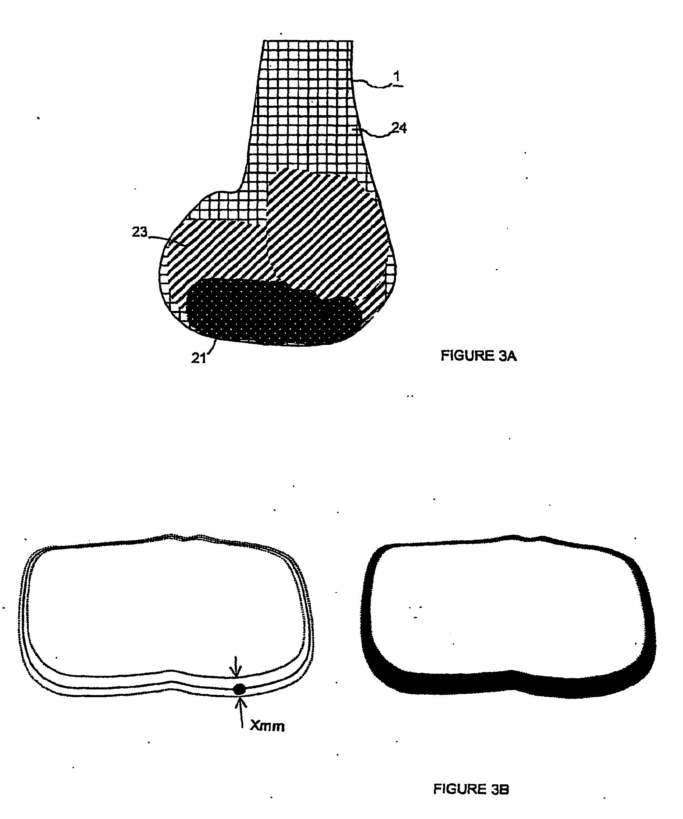

La surface reconstruite par le procédé de déformation ne peut pas être parfaite et des imprécisions subsistent, notamment dans les régions où des points n'ont pas été palpés. Il est important que le chirurgien soit informé des imprécisions de la reconstruction des surfaces. Comme l'illustre la figure 3A, pour donner une indication de cette imprécision au chirurgien, l'invention prévoit d'afficher sur un écran de l'ordinateur les surfaces reconstruites en 3 dimensions avec des colorations de la surface en fonction de l'imprécision estimée. Par exemple, dans les régions 21, critiques pour la prothèse, où de nombreux points ont été palpés, la surface est d'une première couleur. Dans les régions 22 et 23, moins critiques pour la prothèse, où moins de points ont été palpés, la surface est d'une deuxième couleur. Pour le reste du fémur, dans des régions 24 où peu ou pas de palpations auront été effectuées, l'image est sensiblement celle du modèle initial à un facteur d'échelle près et cela est indiqué par une troisième couleur. On peut également coloriser la partie de la surface contenue dans des sphères de X mm autour des points numérisés. On obtient ainsi des zones plus ou moins colorées selon le nombre de palpations. On peut affecter un dégradé de couleurs en fonction des valeurs croissantes de X pour obtenir un effet continu. Les valeurs de l'imprécision de la reconstruction sur la surface peuvent être obtenue par d'autres moyens, l'essentiel étant d'en donner la notion au chirurgien.

Lorsque l'on affiche une coupe plane de la surface reconstruite, on peut encore afficher des couleurs d'imprécision sur les portions des contours d'intersection entre le plan et la surface. De préférence, on affiche des contours autour du contour, d'intersection calculé avec unie épaisseur dépendant tout le long du contour de l'imprécision de la surface estimée dans la région considérée. Comme l'illustre la figure 3B, si un point du contour d'intersection entre le plan et la surface a une imprécision de X mm, on donne une épaisseur de X mm au contour en ce point.

Comme l'illustre la figure 4, il est connu de définir et construire des modèles des structures ligamentaires (incluant aussi tendons, muscles, cartilages et autres) attachées aux surfaces des structures osseuses modélisées. De tels modèles sont des approximations relativement fidèles de la réalité qui permettent de prédire les comportements généraux des structures du genou, en fonction de différentes positions des prothèses, comme cela est décrit dans l'article "A strain-energy model of passive knee kinematics for the study of surgical implantation strategies" de E Chen et al, paru dans Medical Image Computing And Computer-Assisted Intervention - MICCAI'2000, Spinger-Verlag LNCS Series 1935, pages 1086-1095, en octobre 2000. La difficulté principale des méthodes connues réside dans la construction des modèles adaptés à chaque patient. On peut repérer les points d'attache des ligaments sur des images IRM mais cela est imprécis et fastidieux, cela ne donne pas les propriétés élastiques des ligaments et ne traduit pas le comportement global du genou incluant la somme de toutes les structures mineures non repérées. Par souci de simplicité, on considère que l'on a un modèle constitué d'un fémur 1 et d'un tibia 2 reliés entre eux par des ligaments 31, 32, 33. Par exemple, on peut modéliser les ligaments latéraux, les ligaments croisés et les ligaments de la capsule postérieure par de simples droites ou courbes élastiques ou encore des faisceaux de droites et courbes, avec une élongation maximale pour chaque droite, courbe ou fibre. En ayant utilisé la méthode précédemment décrite pour déformer le modèle sur les points de surface numérisés sur l'os réel, on peut appliquer le résultat de la déformation aux points d'attache des structures ligamentaires sur chaque os du modèle de telle sorte qu'ils sont désormais connus dans le repère associé à chaque os. Compte tenu des imprécisions fortes des modèles et des variations fortes de chaque individu, il serait imprécis d'utiliser les paramètres d'élasticité des ligaments du modèle et de les appliquer tels quels aux données du patient. Pour éviter de telles erreurs, au préalable de l'intervention, une fois que les repères de position sont en place dans chaque os, le chirurgien ou autre opérateur exerce des mouvements sur le tibia dans toutes les directions possibles et pour plusieurs flexions du genou jusqu'à atteindre de multiples positions extrêmes du tibia par rapport au fémur. L'opérateur exerce des forces importantes mais raisonnables sur les os, de façon à placer les ligaments proches de leur élongation maximale. L'ordinateur mémorise toutes les positions relatives du tibia par rapport au fémur au cours de ces mouvements. On calcule alors pour chaque ligament reliant un point A du fémur à un point A' du tibia toutes les distances entre les points A et A' pour chacune des positions relatives mémorisées. On ne retient que la distance la plus grande. C'est celle qui correspond à la longueur maximale du ligament. A l'issue de cette étape, chaque modèle de ligament est connu par ses deux points d'attache sur le fémur et sur le tibia, ainsi que par sa longueur maximale lorsqu'il est tendu. A partir de la longueur maximale du ligament en position tendue, et en supposant que la force manuelle imposée au ligament par le chirurgien est à peu près connue, on peut appliquer des modèles connus en biomécanique sur l'extension des ligaments pour leur attribuer une courbe non-linéaire caractérisant l'élongation en fonction de la force de tension, mais un tel modèle très complet ne devra rester que qualitatif et on se contentera en général du modèle des élongations maximales. En pratique, le système selon l'invention comprend des moyens pour mémoriser les extensions maximales des ligaments pour différentes zones angulaires de flexion du genou de façon à compenser les erreurs liées à la position des attaches du ligament dans chacune de ces zones, ces zones angulaires peuvent être la zone de 0 à 10° de flexion, la zone de 10 à 30° de flexion, et la zone de 70 à 120° de flexion. Pour chaque zone on peut aussi se restreindre à reconstruire le modèle des ligaments qui sont connus pour être mis en jeu dans cette zone.

Pour compléter l'acquisition des données nécessaires à la suite, on doit déterminer la position du centre cheville dans le repère du tibia et le centre hanche dans le repère du fémur. Pour ce dernier point on utilise des méthodes connues, par exemple la méthode décrite dans la demande de brevet français FR-A-2785517 (B4181) de P Cinquin et al. intitulé "Procédé et dispositif de détermination du centre d'une articulation". Pour déterminer le centre cheville, l'invention prévoit de numériser des points caractéristiques sur la cheville et de définir le centre cheville par une règle géométrique utilisant ces points caractéristiques, en complément des données acquises sur des radios pré-opératoires. Par exemple, on peut palper les bosses des malléoles interne et externe puis considérer le centre cheville comme le point sur la droite avec une distance relative égale à la distance relative mesurée sur une radio de face de la cheville.

La présente invention propose d'utiliser les divers moyens de mesure et de détermination de position susmentionnés, pour déterminer une position idéale théorique d'implants fémoral et tibial. Il sera clair que l'ordre dans lequel se succèdent les différentes étapes peut être modifié de multiples façon en fonction de chaque technique chirurgicale.

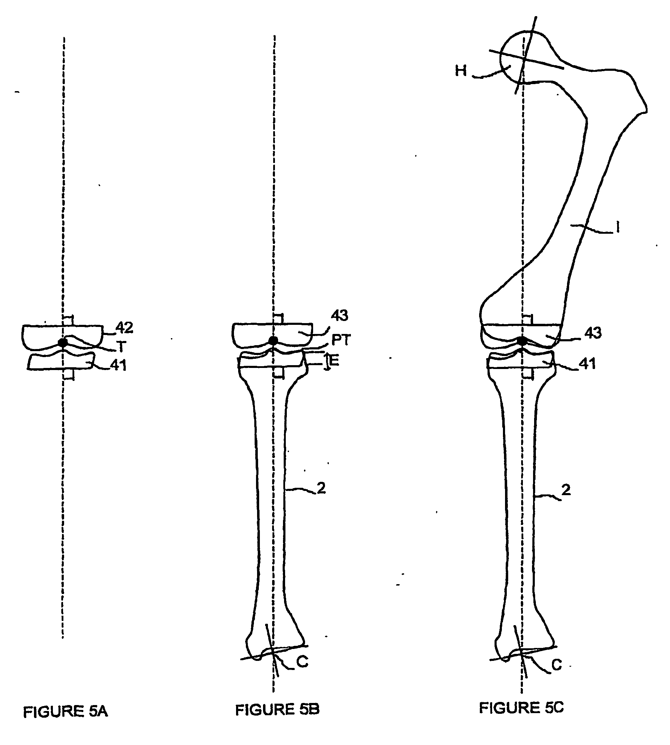

Comme l'illustrent les figures 5A à 5C, on commence par régler la position d'une partie de la prothèse sur un os, par exemple la prothèse tibiale 41. Celle-ci se compose en général d'un plateau tibial fixe ou mobile 42, sa position est définie par les 3 paramètres définissant un plan de coupe et par les 3 paramètres définissant la position de l'implant dans le plan de coupe (2 translations, 1 rotation). On doit également choisir la taille de l'implant parmi une gamme plus ou moins vaste.

Pour commencer, la prothèse tibiale possède un centre T connu mécaniquement par construction de la prothèse. La prothèse tibiale possède également un plan principal qui correspond au plan de coupe sur lequel elle viendra s'appuyer. On impose comme contrainte que la droite passant par le centre prothèse T et perpendiculaire au plan de coupe passe par le centre de la cheville C. Le plan de la prothèse tibiale est alors contraint de se trouver tangent à des sphères centrées sur le centre cheville C. Cette contrainte fixe 2 des 6 paramètres.

Pour continuer, on palpe un point de référence PT sur le tibia et on place le plan de coupe à une distance E du point PT, la valeur de E étant égale à l'épaisseur du plateau tibial. Cette contrainte fixe 1 des 6 paramètres.

Il reste à déterminer la position idéale de l'implant dans le plan de coupe. On commence par déterminer un axe horizontal du genou soit en cherchant l'axe de rotation entre les deux positions de flexion et extension extrêmes du genou, soit en reliant deux points anatomiques particuliers tels que les épicondyles, définis par palpation directe ou définis sur le modèle ajusté au patient. L'angle entre cet axe du genou et l'axe de la prothèse tibiale est établi par défaut à 0°. Les deux paramètres de translation restants sont fixés pour respecter un encombrement géométrique dans chaque plan de coupe considéré.

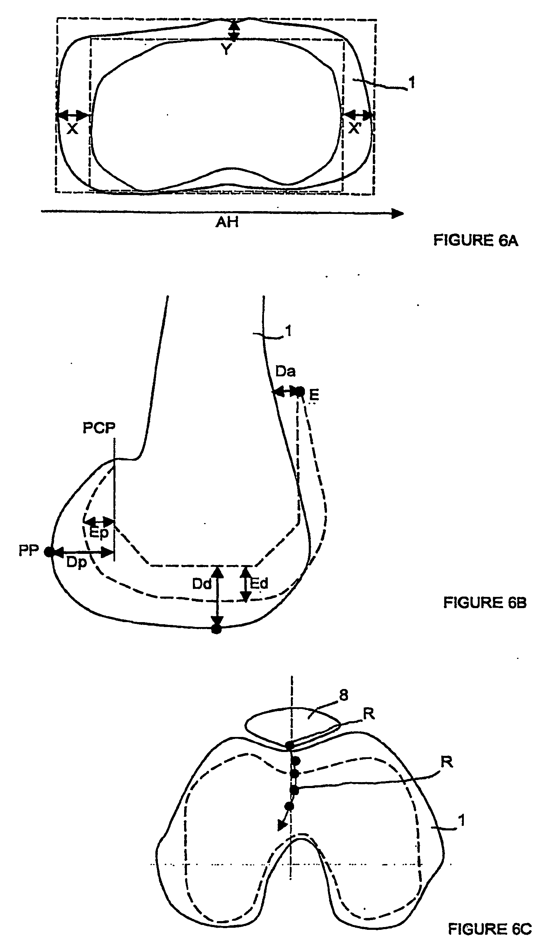

Comme l'illustre la figure 6A, pour la plus petite taille de prothèse donnée, on se donne une position initiale arbitraire de la prothèse, et on calcule les contours d'intersection entre la surface et le plan de coupe correspondant que l'on compare au contour de la prothèse dans ce plan. Pour chacun de ces deux contours, on calcule le rectangle de taille minimale englobant tous les points du contour en imposant une orientation d'un bord du rectangle parallèle à l'axe horizontal AH. Les écarts entre les bords de chacun des deux rectangles sont minimisés, ce qui donne une nouvelle position de la prothèse dans le plan de coupe, et on réitère ce processus jusqu'à sa convergence. On mesure ainsi les valeurs de X et X' entre les bords latéraux de la prothèse et les bords latéraux du contour de la coupe sur la surface et on ajuste la position latérale de façon à égaliser X et X'. De la même façon, on règle la distance d'avant en arrière Y de telle sorte que la prothèse vienne à Y min du bord antérieur du contour d'intersection, Y étant une valeur fixée par défaut par le chirurgien. On réitère tout ce processus en cherchant la plus grande taille pour laquelle tous les bords de la prothèse sont à l'intérieur des bords du contour d'intersection, y compris les bords postérieurs. La position, la taille et l'orientation de la prothèse tibiale sont ainsi totalement déterminées automatiquement. Si besoin, l'opérateur peut alors bouger chacun des paramètres, à l'aide d'un écran tactile de préférence (ou tout autre équivalent de souris), pour s'aligner sur des valeurs de son choix en fonction de chaque recommandation de technique chirurgicale.

On effectue une détermination de la position de la prothèse fémorale de façon à peu près équivalente en tenant compte du centre H de la hanche à la place du centre de la cheville. Comme l'illustre la figure 6B, la position antéro-postérieure de la prothèse est calculée automatiquement pour égaliser l'épaisseur Ep du condyle postérieur de la prothèse avec la distance Dp calculée entre le plan de coupe postérieur de la prothèse PCP et le point le plus postérieur PP de la surface d'un des deux condyles postérieur du genou choisi par le chirurgien. Ce point postérieur PP est déterminé automatiquement par l'ordinateur comme étant le point du condyle considéré sur le modèle et adapté au patient ayant la coordonnée Y la plus petite dans la direction d'un vecteur Y allant du postérieur vers l'antérieur. Ce principe est applicable à la recherche de tous les points extrêmes dans une certaine direction. La taille de la prothèse la plus grande est déterminée par l'ordinateur pour que la distance entre le point extrême E le plus proximal de la prothèse soit le plus proche de la surface du fémur tout en restant à l'intérieur de cette surface. Ce critère est réalisé en minimisant itérativement la distance entre le point extrême de la prothèse mémorisé et toutes les facettes triangulaires composant la surface. La position distale de la prothèse est déterminée par l'ordinateur pour égaliser l'épaisseur du condyle distal de la prothèse Ep avec la distance Dp calculée entre le plan de coupe distal de la prothèse et le point le plus distal de la surface d'un des deux condyles distaux du genou choisi par le chirurgien.

Pour régler la position latérale de la prothèse du fémur, on peut utiliser les bords latéraux comme cela a été décrit pour le tibia. De façon préférentielle, l'invention propose d'enregistrer la trajectoire d'un point milieu R de la surface de la rotule au cours d'un mouvement de flexion du genou, en commençant par l'extension complète. On utilise pour cela un repère de position petit et léger fixé sur la partie externe de la rotule à l'aide de petites broches fines et peu profondes. Pour une position de la rotule 8 refermée sur le fémur 1 (obtenue par exemple au début de l'opération), on enregistre les mouvements du repère de la rotule par rapport au repère du fémur. Le point R décrit donc une trajectoire qui est connue dans le repère du fémur. La prothèse du fémur contient une échancrure pour accueillir la rotule au cours de la flexion du genou. Le milieu de cette échancrure est dans un plan.

Comme l'illustre la figure 6C, on détermine la position de la prothèse du fémur de façon à faire coïncider au mieux le plan de l'échancrure avec la trajectoire du point R. Eventuellement on peut utiliser un paramètre de rotation pour optimiser cet alignement. Pour optimiser tous ces critères en même temps, on part d'une position initiale arbitraire déterminée empiriquement proche de la solution recherchée, puis on minimise les critères un par un en modifiant la valeur du paramètre de position, orientation ou taille de la prothèse qui l'influence le plus, de façon itérative, jusqu'à obtenir une convergence stable de la position, orientation et taille idéale de l'implant. Lorsque plusieurs paramètres influencent un critère, on a recours à des méthodes d'optimisation multidimensionnelle bien connues dans la littérature mathématique.

Jusqu'ici, on a supposé que les positions du tibia et du fémur étaient indépendantes. En réalité, les prothèses du fémur et du tibia sont conçues en définissant une trajectoire idéale de la prothèse tibiale sur la prothèse fémorale de la flexion à l'extension, au moins pour 3 ou 4 positions de flexion, par exemple à 0°, 30°, 90° et 120°. Pour chacun de ces angles, la position relative idéale entre le composant fémoral et le composant tibial de la prothèse est totalement déterminée. Certaines prothèses sont dites non-congruentes et autorisent des mouvements plus complexes qu'une simple trajectoire de flexion du composant tibial par rapport au composant fémoral, mais la cinématique moyenne et neutre décrit de façon significative un comportement idéal recherché, les écarts par rapport à cette cinématique neutre pouvant par ailleurs aussi être pris en compte par le procédé mis en oeuvre par la présente invention. A ce stade, on peut donc simuler l'ensemble de toutes les positions relatives des implants et des os pour différents mouvements choisis en fonction de la prothèse considérée. Le modèle permet alors de prédire que les ligaments ne dépassent pas leurs valeurs limite pour toutes ces positions relatives. Si certains ligaments dépassent leurs limites, le chirurgien pourra utiliser cette information pour prévoir de détendre chirurgicalement le ligament. A tout moment, le chirurgien peut intervenir sur les ligaments et de nouveau acquérir les positions extrêmes de façon aléatoire pour recalibrer les paramètres des ligaments et recommencer la prédiction.

Pour toute position globale, la présente invention prévoit de simuler des mouvements relatifs du tibia et du fémur par des rotations autour des points de contact existant sur les condyles de façon à simuler des positions dans lesquelles les ligaments sont en élongation maximale lorsque l'on applique des forces à peu près équivalentes en intensité et direction aux forces appliquées lors de la mesure des élongations maximales des ligaments. On peut ainsi de prédire la balance ligamentaire pour différents angles de flexion caractérisée par l'amplitude des mouvements et par la dissymétrie des mouvements possibles autour de la position neutre.

Le chirurgien peut alors déterminer librement les paramètres essentiels qui influent le plus sur le réglage de la balance ligamentaire, sans avoir fait encore aucune coupe osseuse. Il peut par exemple choisir de conserver un alignement parfait des centres hanche et cheville avec le centre prothèse en extension, mais en inclinant la ligne perpendiculaire aux plans de coupe par rapport à l'axe mécanique, créant ainsi des interlignes obliques entre les prothèses. Tout type de technique chirurgicale peut ainsi être simulé en utilisant le système selon l'invention.

Une fois que l'on a déterminé chaque plan de coupe d'un os (tibia ou fémur), on prévoit de fixer à cet os un guide de coupe dans lequel on vient engager une lame de scie pour effectuer la coupe avec précision selon l'angle déterminé par l'ordinateur (éventuellement retouché par le chirurgien). Un guide de coupe comprend en général au moins deux canons cylindriques permettant de placer des mèches fixées dans l'os. Un guide de coupe est un instrument relativement encombrant et lourd, il faut en général utiliser des supports réglables avec des vis, des molettes ou des cales pour tenir et placer ces guides de coupe, ces supports étant eux-mêmes fixés au patient par diverses tiges lourdes, encombrantes et souvent invasives, c'est-à-dire détériorant l'os de façon significative dans ses parties saines. Idéalement, on souhaiterait se passer de tels supports et placer les guides de coupe directement mais cet acte est très difficile à réaliser manuellement. L'alignement direct d'un plan sur un plan idéal ou d'un solide sur un solide idéal est une opération délicate, même en utilisant des alignements visuels sur un écran qui montre les positions réelles et visées de chaque structure selon divers modes de représentation graphiques. La présente invention, au lieu d'essayer de positionner directement un guide de coupe, prévoit d'utiliser au préalable un guide de perçage comprenant deux canons cylindriques ayant exactement le même écartement que ceux du guide de coupe et un picot ponctuel servant de contrainte mécanique.

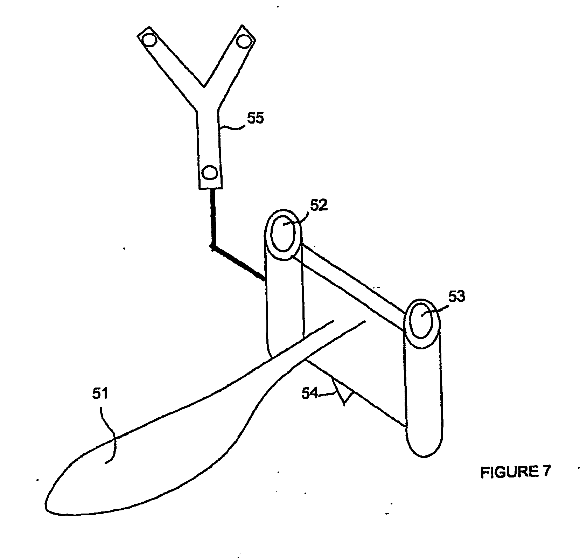

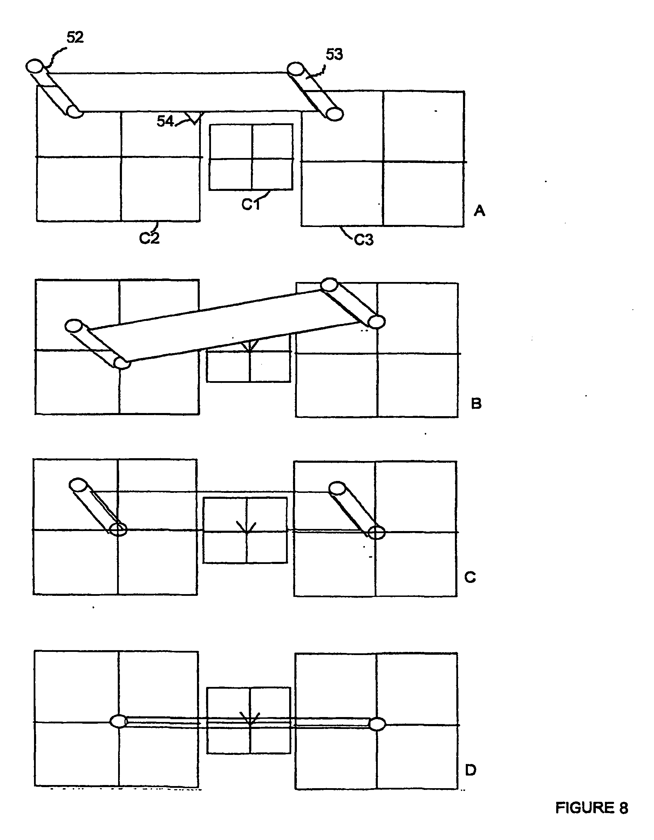

La figure 7 représente un guide de perçage selon un mode de réalisation de la présente invention. Ce guide de perçage comporte un manche 51. Les deux canons cylindriques sont désignés par les références 52 et 53. A ce guide de perçage est associé rigidement un repère de position 55 visible par le système de positionnement tridimensionnel surveillant la scène d'opération. Le repère 55 est par exemple muni de 3 marqueurs réfléchissants et est en forme de Y. Selon une caractéristique importante de l'invention, le guide de percage comprend un picot pointu 54. La forme générale de ce guide de perçage, à savoir la position relative du picot 54 et des canons 52 et 53 dans le repère de position en Y est mémorisée dans l'ordinateur utilisé pour déterminer la position et l'orientation du plan de coupe par rapport à l'os considéré. Comme le représente la figure 8, cet ordinateur assure l'affichage d'une cible comprenant une cible centrale C1 et des cibles latérales C2 et C3. L'affichage de la cible C1 est une vue en perspective parallèle dans la direction de l'axe du picot. L'affichage des cibles C2 et C3 sont des vues en perspective parallèle dans la direction de l'axe des canons. En premier lieu, l'image du picot 54 doit être amenée exactement sur le centre de la cible C1. Il faut pour cela glisser le picot sur la surface de l'os selon deux translations, ce qui est facile. Une fois la bonne position du picot trouvée, on plante très légèrement le picot dans l'os de façon à ce qu'il reste stable par la suite. Tous les mouvements du guide sont alors contraints à tourner autour de ce point fixe. Les images des canons 52 et 53 sont alors amenées sur les cibles latérales C2 et C3, ce qui se fait facilement en réglant trois rotations autour d'un point fixe et stable mécaniquement. Ces opérations sont effectuées par le chirurgien ou autre opérateur en déplaçant le guide de perçage sur l'os et en surveillant son image à l'ordinateur qui apparaît sensiblement comme cela est représenté en figure 8A. Ainsi, à l'étape de la figure 8B le picot 54 est mis en contact avec la cible centrale 61. A l'étape de la figure 8C les canons 52 et 53 sont amenés en regard des cibles C2 et C3 et, à l'étape de la figure 8D, les canons sont placés selon l'orientation verticale convenable. Une fois le guide de perçage en position, on fixe des mèches insérées dans les canons 52 et 53 dans l'os.

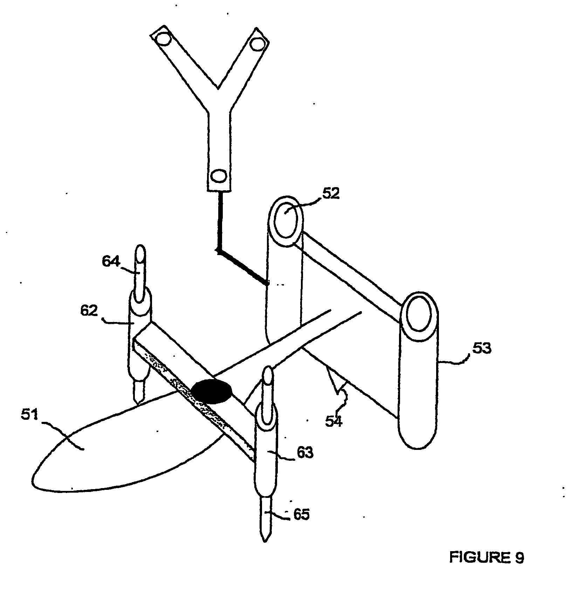

La figure 9 représente une variante du guide de perçage de la figure 7 munie de moyens de blocage en position pour éviter tout glissement lors de la mise en place des mèches dans l'os. Pour cela, on monte sur le manche 51 un bâti horizontal 61 qui porte à sets deux extrémités, de part et d'autre du manche 51, deux canons verticaux auxiliaires 62 et 63. Chacun des canons 62, 63 reçoit une tige pointue, respectivement 64, 65 qui traverse ce canon. A l'intérieur du bâti 61 est prévu un moyen de blocage actionné par un bouton 67. Un tel blocage peut être réalisé par un ressort qui maintient une pression sur les tiges. Quand le bouton est pressé les tiges 64, 65 coulissent librement dans les canons 62, 63. Ainsi, elles descendent jusqu'à buter contre l'os au-dessus duquel elles sont placées. Dès que le bouton 67 est relâché, les tiges sont bloquées en position. Le chirurgien, dès qu'il est arrivé à la position illustrée en figure 8D, peut alors sans avoir à continuer à surveiller la cible apparaissant sur l'écran de l'ordinateur, monter les mèches en étant assuré que le guide de perçage reste bien en position, puisqu'il est en appui sur 3 points fixes.

Une fois que les mèches de perçage sont positionnées dans l'os, on enlève le guide de perçage et on monte le premier guide de coupe sur les mèches qui viennent d'être posées. Pour une prothèse telle que le fémur qui comporte plusieurs plans de coupe, on peut ensuite adjoindre une série de guides de coupe calibrés pour réaliser l'ensemble des coupes correspondant aux plans d'appuis de la prothèse, en venant se caler sur ces deux mèches de référence placées dans l'os. Pour chacun de ces guides de coupe, la position relative des deux canons destinés à contenir les mèches de maintien et les plans de coupes est connue de façon précise. Il est important de noter à cet égard que le picot impose un degré de liberté des guides de coupe puisqu'il est en contact avec la surface. Le choix de la position du picot est donc très importante puisqu'elle conditionne la position finale de la prothèse.

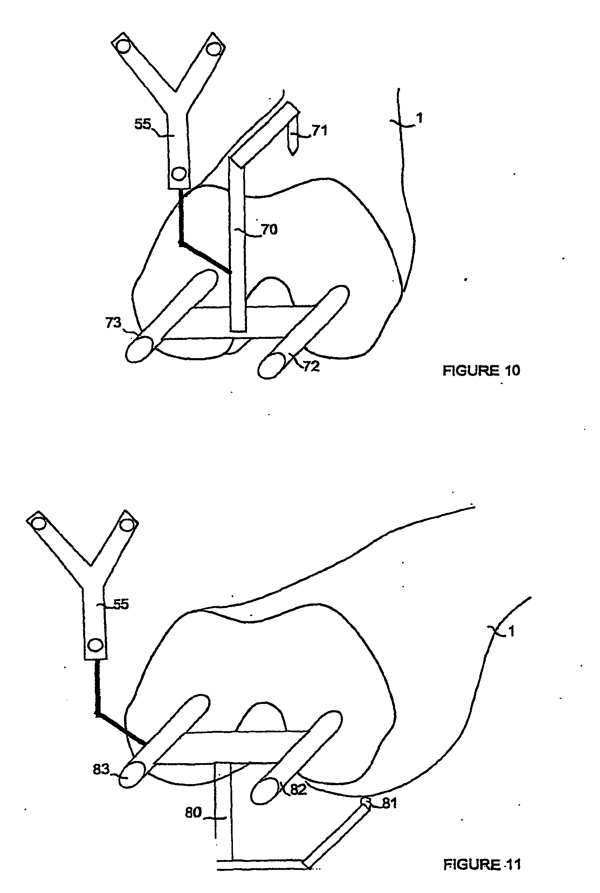

La figure 10 représente un autre guide de perçage 70 applicable spécifiquement à la prothèse du fémur dans lequel le picot de maintien 71 est déporté par rapport aux canons de perçage 72, 73 pour venir s'appuyer sur la surface antérieure du fémur 1, de façon à contraindre mécaniquement le guide de perçage à respecter la position antérieure de la future prothèse avec précision.

La figure 11 représente un autre guide de perçage 80 applicable spécifiquement à la prothèse du fémur dans lequel le picot de maintien 81 est déporté par rapport aux canons de perçage 82, 83 pour venir s'appuyer sur la surface postérieure d'un des deux condyles du fémur 1, de façon à contraindre mécaniquement le guide de perçage à respecter la position postérieure de la future prothèse avec précision.

Bien entendu, cette structure est susceptible de nombreuses variantes et modifications qui apparaîtront à l'homme du métier. Par exemple, on pourrait prévoir plusieurs picots et une seule tige de blocage. Tous les guides de perçage décrits dans la présente invention seront réalisés dans des formes et matériaux pour être le plus léger possible, de façon à être facilement manipulables.

Une fois que l'on a réalisé un certain nombre de plans de coupe, on peut introduire des cales entre le fémur et le tibia en appui sur ces coupes au niveau des deux condyles du fémur pour vérifier que l'équilibrage ligamentaire sera correct une fois les prothèses mises en place. Classiquement, le chirurgien déplace manuellement le tibia de gauche à droite pour évaluer son jeu angulaire, mais cette mesure reste imprécise. Le système selon la présente invention permet de mesurer la variation angulaire avec précision et de l'afficher sur l'écran de l'ordinateur. On affiche les deux angles Bd et Bg signés obtenus par un déplacement extrême vers la gauche et vers la droite par rapport à la position neutre du genou au repos. La différence entre les valeurs absolues des deux angles Bd et Bg est une mesure du déséquilibre ligamentaire de gauche à droite. La somme des valeurs absolues des angles Bd et Bg est une mesure de la tension ligamentaire globale. Ces indicateurs Bd+Bg et Bd-Bg sont mesurés et affichés pour divers angles de flexion. De façon préférentielle, ces angles Bd et Bg sont représentés graphiquement sur un écran par un segment de droite vertical correspondant à la position neutre et par deux segments intersectant le segment neutre en son sommet et faisant des valeurs angulaires de Bd et Bg avec le segment neutre. A l'aide de ces mesures, le chirurgien peut tendre ou détendre les ligaments, ou encore additionner ou soustraire des épaisseurs entre les prothèses du fémur et du tibia pour obtenir un bon équilibre pour tous les degrés de flexion.

Selon un autre mode de réalisation préférentiel, l'invention prévoit d'afficher les distances entre les points les plus proches se faisant face sur le fémur et le tibia équipés de leur prothèse simulée ou réelle, pour chacun des deux condyles, et pour plusieurs angles de flexion, lorsque le genou est soumis à des contraintes externes de balance de gauche à droite.

Une fois que la prothèse fémorale est en place, de nombreuses techniques chirurgicales invitent à placer une petite prothèse de type bouton sur la face interne de la rotule. Le système selon la présente invention permet de déterminer la position idéale de ce bouton rotulien. La position de la gorge de la prothèse fémorale peut être déterminée dans le repère de position associé au fémur, soit en utilisant la position prédite précédemment, soit en numérisant in fine la gorge par palpation sur la prothèse posée. Les positions relatives du repère de position fixé à la rotule sont connues et mémorisées pour plusieurs angles de flexion. Pour une position choisie de flexion, on peut alors afficher à l'écran la position de la gorge numérisée en la projetant sur une vue correspondant à la face interne de la rotule. Ou inversement, on peut projeter la trajectoire de la rotule obtenue pour toutes les flexions selon une vue axiale ou frontale de la trochlée du fémur. Après avoir coupé la rotule en épaisseur de façon classique, le chirurgien peut ensuite utiliser un guide de perçage et viser un point sur la face interne de la rotule qui coïncide avec la gorge sur le fémur, soit globalement, soit pour une zone angulaire de flexion donnée, de façon à ce que la rotule pénètre bien au milieu de la gorge vers 20 degrés de flexion, et de façon à équilibrer les forces latérales qui seront exercées entre la rotule et la prothèse fémorale. Ce perçage dans l'os servira ensuite de centrage pour placer le bouton rotulien final.

Le système décrit dans cette invention peut être appliqué à tout type de prothèse du genou et il est compatible avec la plupart des techniques chirurgicales de pose de prothèse du genou, il peut être étendu à d'autres articulations telles que le coude ou l'épaule.

## Revendications

1. Système automatique de détermination de la position théorique idéale d'une prothèse du genou comprenant, pour la détermination de la position d'une prothèse tibiale, des moyens adaptés à :
déterminer la forme de la partie proximale du tibia et sa position par rapport au entre de l'articulation de la cheville ;
déterminer par l'utilisateur un point haut de référence du plateau tibial ;
calculer de manière itérative la position, l'orientation et la taille par rapport au tibia de la prothèse tibiale et du plan de coupe correspondant à la prothèse tibiale en tenant compte des contraintes suivantes :
- la perpendiculaire au plan de coupe passant par le centre de la prothèse passe aussi par le centre de l'articulation de la cheville,
- le plan de coupe est à une distance dudit point haut de référence égale à l'épaisseur de la prothèse à fixer,
- le grand côté de la prothèse est centré sur le grand côté de la section du tibia dans le plan de coupe,
- le bord antérieur du petit côté de la prothèse est à une distance prédéterminée du bord antérieur du petit côté de la section du tibia dans le plan de coupe ; et des moyens adaptés à
déterminer l'orientation dans le plan de coupe de la prothèse de façon que le grand côté de la prothèse soit parallèle à l'axe horizontal du genou.

2. Système selon la revendication 1, comprenant en outre, pour la détermination de la position d'une prothèse fémorale, des moyens adaptés à :
déterminer la position relative de la partie distale du fémur par rapport au centre de l'articulation de la hanche ;
calculer la position, l'orientation et la taille par rapport au fémur de la prothèse fémorale et du plan de coupe correspondant à la prothèse fémorale en tenant compte des contraintes suivantes :
- la perpendiculaire au plan de coupe passant par le centre de la prothèse passe aussi par le centre de l'articulation de la hanche,
- le plan de coupe distal est à une distance du point le plus distal sur un des condyles égale à l'épaisseur de la prothèse à fixer,
- le plan de coupe postérieure est à une distance du point le plus postérieur sur un des condyles égale à l'épaisseur de la prothèse à fixer,
- le grand côté de la prothèse est centré sur le grand côté de la section du fémur dans le plan de coupe,
- la taille maximale de la prothèse est telle que le bord de la prothèse est le plus proche possible mais à l'intérieur de la surface du fémur; et des moyens adaptés à
déterminer l'orientation dans le plan de coupe de la prothèse de façon que le grand côté de la prothèse soit parallèle à l'axe horizontal du genou.

3. Système selon la revendication 2, **caractérisé en ce que** l'orientation et la position latérale de la prothèse fémorale sont ajustées par un alignement du plan de la trochlée de la prothèse fémorale avec la trajectoire du centre de la rotule numérisée au cours d'un mouvement de flexion de genou grâce à un repère de position placé sur la face externe de la roule.

4. Système selon la revendication 2, **caractérisé en ce qu'**il comprend en outre, pour la détermination des formes et positions du tibia, du fémur, du centre de l'articulation de la cheville et du centre de l'articulation de la hanche :
des éléments repérables en position dans un système tridimensionnel de positionnement, fixables au moins au tibia et au fémur,
un palpeur, un dispositif échographique, et/ou un dispositif radiographique pour repérer dans ledit système tridimensionnel de positionnement la position de divers points du tibia et du fémur par rapport auxdits éléments repérables, et
des moyens adaptés à déformer et ajuster un modèle préétabli des os de la jambe en utilisant les informations de position recueillies.

5. Système selon la revendication 4, **caractérisé en ce que** ledit modèle comprend également une modélisation des positions des attaches des ligaments de l'articulation du genou, **en ce que** cette modélisation est adaptée au patient en même temps que le modèle de la surface osseuse, et **en ce que** ledit modèle comprend également les valeurs des élongations maximales de chaque ligament, déterminées expérimentalement en faisant bouger la jambe du patient non encore opérée pour repérer les limites de déplacement liées aux ligaments existants.

6. Système selon la revendication 5, comprenant en outre des moyens adaptés à :
simuler sur l'image de l'ensemble fémur-tibia la mise en position des prothèses liées entre elles pour différentes positions de flexion, et la position des ligaments du genou ;
en déduire les limites de mouvement qu'aura la jambe munie des prothèses avec les ligaments existants ; et
tenir compte de cette information pour modifier la position théorique idéale de la prothèse du genou, et/ou pour suggérer une intervention sur les ligaments.

7. Système selon la revendication 4, **caractérisé en ce que** ledit palpeur a une extrémité en forme de boule et **en ce que** les repérages par le palpeur sont effectués de façon dynamique tandis que l'on déplace ladite boule contre la partie à analyser, le système de positionnement tridimensionnel étant conçu pour déterminer les positions instantanées du centre de la boule et un système de reconstitution de surface à partir d'un modèle déformable étant conçu pour effectuer une correction correspondant au rayon de la boule.

8. Système selon la revendication 4, **caractérisé en ce que** l'on forme une image du modèle ajusté et **en ce qu'**on affiche cette image en affectant ses diverses zones de couleurs ou d'épaisseur caractérisant la densité de points relevée dans cette zone par le palpeur, ce qui indique le degré de précision du modèle de la zone correspondante.

9. Système selon la revendication 4, comprenant des moyens adaptés à :
déterminer par le calcul des emplacements auxquels le tibia/fémur doit être percé pour mettre en place un guide de coupe,
pointer ces emplacements sur l'image affichée du tibia/fémur,
afficher en permanence sur l'image reconstituée du tibia/fémur, l'image d'un guide de perçage muni de moyens de repérage dans ledit système tridimensionnel de positionnement, ce guide comportant des tubes de même écartement que des tubes du guide de coupe destinés à être montés sur des mèches fixées dans l'os,
**caractérisé en ce que** le guide de perçage comprend un picot central pouvant être mis en appui sur l'os et dont l'image doit coïncider avec un point cible prépositionné sur l'image affichée du tibia/fémur.

10. Système selon la revendication 9, **caractérisé en ce que** le guide de perçage comprend en outre des moyens de calage, actionnables une fois que le guide a été amené en position.

11. Système selon la revendication 4, **caractérisé en ce que** les éléments repérables respectivement associés au tibia, au fémur et au palpeur sont géométriquement différentiés..

12. Système selon la revendication 11, **caractérisé en ce que** l'élément repérable associé au tibia a la forme de la lettre T, l'élément repérable associé au fémur a la forme de la lettre F, l'élément repérable associé au palpeur a la forme de la lettre P.

13. Système selon la revendication 2, **caractérisé en ce que** la position du composant rotulien de la prothèse est obtenue par des moyens adaptés à :
déterminer dans un système tridimensionnel de positionnement, la trajectoire d'un élément repérable en position fixé à la face externe de la rotule au cours d'un mouvement de flexion du genou,
déterminer le point de la face interne de la rotule qui coïncide le mieux avec le centre de la gorge de la prothèse du fémur pour une zone angulaire de flexion choisie,
guider le placement d'une mèche de perçage vers ledit point.

## Claims

1. An automatic system for determining the ideal theoretical position of a knee prosthesis including, for the determination of the position of a tibial prosthesis, means adapted to:
determining the shape of the proximal portion of the tibia and its position with respect to the center of the ankle joint;
determining by the user a reference high point of the superior surface of the tibia;
iteratively calculating the position, the orientation, and the size with respect to the tibia of the tibial prosthesis and of the section plane corresponding to the tibial prosthesis, taking the following constraints into account:
- the perpendicular to the section plane crossing the center of the prosthesis also crosses the center of the ankle joint,
- the section plane is at a distance from said reference high point equal to the thickness of the prosthesis to be attached,
- the large side of the prosthesis is centered on the large side of the section of the tibia in the section plane,
- the anterior edge of the small side of the prosthesis is at a predetermined distance from the anterior edge of the small side of the tibia section in the section plane; and
determining the orientation in the section plane of the prosthesis so that the large side of the prosthesis is parallel to the horizontal axis of the knee.

2. The system of claim 1, further including, to determine the position of a femoral prosthesis, means adapted to:
determining the relative position of the distal portion of the femur with respect to the center of the hip joint;
calculating the position, the orientation and the size with respect to the femur of the femoral prosthesis and of the section plane corresponding to the femoral prosthesis, taking the following constraints into account:
- the perpendicular to the section plane crossing the center of the prosthesis also crosses the center of the hip joint,
- the distal section plane is at a distance from the most distal point on one of the condyles equal to the thickness of the prosthesis to be attached,
- the posterior section plane is at a distance from the most posterior point on one of the condyles equal to the thickness of the prosthesis to be attached,
- the large side of the prosthesis is centered on the large side of the section of the femur in the section plane,
- the maximum size of the prosthesis is such that the edge of the prosthesis is as close as possible but inside of the femur surface; and
determining the orientation in the section plane of the prosthesis so that the large side of the prosthesis is parallel to the horizontal axis of the knee.

3. The system of claim 2, **characterized in that** the orientation and the lateral position of the femoral prosthesis are adjusted with an alignment of the plane of the trochlea of the femoral prosthesis with the trajectory of the center of the digitized patella during a knee flexion motion by means of a position mark placed on the external surface of the patella.

4. The system of claim 2, **characterized in that** it further includes, to determine the shapes and positions of the tibia, of the femur, of the center of the ankle joint and of the center of the hip joint:
elements locatable in position in a tridimensional positioning system, attachable at least to the tibia and to the femur,
a palpation device, an echographic device, and/or a radiographic device to locate in said tridimensional positioning system the position of various points of the tibia and of the femur with respect to said locatable elements, and
means adapted to adjusting a preestablished model of the leg bones by using the collected position information.

5. The system of claim 4, **characterized in that** said model also includes a modeling of the positions of the attachments of the ligaments of the knee joint, this modeling being adapted to the patient at the same time as the model of the bone surface, and **in that** said model also includes the values of the maximum elongations of each ligament, experimentally determined by having the still non-operated patient's leg move to locate the displacement limits linked to existing ligaments.

6. The system of claim 5, further including means adapted to:
simulating on the image of the femur-tibia assembly the positioning of the prostheses linked together for different flexion positions, and the position of the knee ligaments;
deducing therefrom the motion limits that the leg provided with the prosthesis will have with the existing ligaments; and
taking this information into account to modify the theoretical ideal position of the knee prosthesis, and/or to suggest an intervention on the ligaments.

7. The system of claim 4, **characterized in that** said palpation device has a ball-shaped end and the locating by the palpation device is performed dynamically as said ball is displaced against the portion to be analyzed, the tridimensional positioning system being designed to determine the instantaneous positions of the ball center and a system of surface reconstitution from a deformable model being designed to perform a correction corresponding to the ball radius.

8. The system of claim 4, **characterized in that** an image of the adjusted model is formed and **in that** this image is displayed by assigning its various areas colors or thicknesses characterizing the density of points found in this area by the palpation device, which indicates the degree of accuracy of the model of the corresponding area.

9. The system of claim 4, including means adapted to:
determining by calculation locations at which the tibia/femur must be drilled into to accommodate a section guide;
pointing these locations on the displayed image of the tibia/femur,
permanently displaying on the restored image of the tibia/femur the image of a drilling guide provided with locating means in said tridimensional positioning system, this guide including tubes separated by the same interval as tubes of the section guide intended to be assembled on threads fixed in the bone,
**characterized in that** the drilling guide includes a central point that can bear against the bone and the image of which must coincide with a target point pre-positioned on the displayed image of the tibia/femur.

10. The system of claim 9, **characterized in that** the drilling guide further includes adjustment means, operable once the guide has been brought to its position.

11. The system of claim 4, **characterized in that** the locatable elements respectively associated with the tibia, with the femur and with the sensor are geometrically differentiated.

12. The system of claim 11, **characterized in that** the locatable element associated with the tibia has the shape of letter T, the locatable element associated with the femur has the shape of letter F, the locatable element associated with the sensor has the shape of letter P.

13. The system of claim 2, **characterized in that** the position of the patella component of the prosthesis is obtained by means adapted to:
determining in a tridimensional positioning system the trajectory of an element having a locatable position attached to the external surface of the patella in a knee flexion motion,
determining the point of the internal patella surface which better coincides with the center of the groove of the prosthesis of the femur for a selected angular flexion area,
guiding the placing of a drilling thread towards said point.

## Patentansprüche

1. Automatisches System zum Bestimmen der idealen theoretischen Position einer Knieprothese mit Mitteln, die zum Bestimmen der Position einer schienbeinseitigen Prothese eingerichtet sind zum:
Bestimmen der Form des proximalen Teiles des Schienbeins und seiner Position bezüglich des Mittelpunkts des Fußgelenks;
Bestimmen eines Bezugshochpunkts der oberen Oberfläche des Schienbeins durch den Benutzer;
iterativen Berechnen der Position, der Orientierung und der Größe bezüglich des Schienbeins der schienbeinseitigen Prothese und der der schienbeinseitigen Prothese entsprechenden Schnittebene unter Beachtung der nachfolgenden Bedingungen:
- die Senkrechte zu der Schnittebene, welche den Mittelpunkt der Prothese schneidet, schneidet auch den Mittelpunkt des Fußgelenks,
- der Abstand der Schnittebene von dem Bezugshochpunkt stimmt mit der Dicke der anzubringenden Prothese überein,
- das breite Ende der Prothese ist zentriert auf das breite Ende des Abschnittes des Schienbeins in der Schnittebene,
- die Vorderkante des schmalen Endes der Prothese liegt in einem vorbestimmten Abstand von der vorderen Kante des schmalen Endes des Schienbeinabschnittes in der Schnittebene; und
Bestimmen der Orientierung in der Schnittebene der Prothese, so dass das breite Ende der Prothese parallel zu der Horizontalachse des Knies verläuft.

2. System nach Anspruch 1 mit Mitteln, welche zum Bestimmen der Position einer oberschenkelseitigen Prothese eingerichtet sind zum:
Bestimmen der relativen Position des distalen Teils des Oberschenkelknochens bezüglich des Mittelpunkts des Hüftgelenks;
Berechnen der Position, der Orientierung und der Größe bezüglich des Oberschenkelknochens der oberschenkelseitigen Prothese und der der oberschenkelseitigen Prothese entsprechenden Schnittebene unter Beachtung der nachfolgenden Bedingungen:
- die Senkrechte zu der Schnittebene, welche den Mittelpunkt der Prothese schneidet, schneidet auch den Mittelpunkt des Hüftgelenks,
- der Abstand der distalen Schnittebene von dem am weitesten distalen Punkt auf einem der Kondylen stimmt mit der Dicke der anzubringenden Prothese überein,
- der Abstand der hinteren Schnittebene von dem am weitesten hintenliegenden Punkt auf einem der Kondylen stimmt mit der Dicke der anzubringenden Prothese überein,
- das breite Ende der Prothese ist zentriert auf das breite Ende des Abschnittes des Oberschenkelknochens in der Schnittebene,
- die Maximalgröße der Prothese ist derart, dass die Kante der Prothese so nah wie möglich an der Oberfläche des Oberschenkelknochens, aber innerhalb der Oberfläche des Oberschenkelknochens, liegt; und
Bestimmen der Orientierung in der Schnittebene der Prothese, so dass das breite Ende der Prothese parallel zu der Horizontalachse des Knies verläuft.

3. System nach Anspruch 2, **dadurch gekennzeichnet, dass** die Orientierung und die seitliche Position der oberschenkelseitigen Prothese mit einer Ausrichtung der Ebene der Trochlea der oberschenkelseitigen Prothese zu der Bewegungslinie des Mittelpunkts der digitalisierten Kniescheibe während einer Kniebeugebewegung mittels einer Positionsmarkierung, welche auf der externen Oberfläche der Kniescheibe angebracht ist, angepasst werden.

4. System nach Anspruch 2 mit zusätzlichen Mitteln zum Bestimmen der Formen und Positionen des Schienbeins, des Oberschenkelknochens, des Mittelpunktes des Fußgelenkes und des Mittelpunktes des Hüftgelenkes, umfassend:
Elemente, welche in einem dreidimensionalen Positionierungssystem lokalisierbar sind und wenigstens mit dem Schienbein und mit dem Oberschenkelknochen verbindbar sind,
eine Abtastvorrichtung, eine Echographievorrichtung und/oder eine Radiographievorrichtung zum Bestimmen der Position verschiedener Punkte des Schienbeins und des Oberschenkelknochens bezüglich der lokalisierbaren Elemente in dem dreidimensionalen Positionierungssystem, und
Mittel, welche zum Anpassen eines vorher festgelegten Modelles der Beinknochen unter Verwendung der gesammelten Positionsinformation eingerichtet sind.

5. System nach Anspruch 4, **dadurch gekennzeichnet, dass** das Modell auch eine Modellierung der Positionen der Befestigungen der Bänder des Kniegelenkes umfasst, wobei diese Modellierung gleichzeitig mit dem Modell der Knochenoberfläche an den Patienten angepasst wird, sowie ferner **gekennzeichnet dadurch, dass** das Modell die Werte der Maximalauslenkung jedes Bandes umfasst, welche experimentell **dadurch** bestimmt werden, dass das noch unoperierte Bein des Patienten bewegt wird, um die mit den bestehenden Bändern verknüpften Auslenkungsgrenzen zu bestimmen.

6. System nach Anspruch 5 mit Mitteln, die eingerichtet sind zum:
Simulieren der Positionierung der miteinander verbundenen Prothesen für unterschiedliche Beugungspositionen und der Position der Kniebänder auf dem Bild der Oberschenkel-Schienb ein-Gruppe;
Bestimmen der Bewegungsgrenzen, welches das mit der Prothese ausgestattete Bein mit den bestehenden Bändern aufweisen wird, auf Grundlage der Simulation; und
Berücksichtigen dieser Informationen zum Modifizieren der theoretischen Idealposition der Knieprothese und/oder zum Vorschlagen eines Eingriffs an den Bändern.

7. System nach Anspruch 4, **dadurch gekennzeichnet, dass** die Abtastvorrichtung ein kugelförmiges Ende aufweist und die Positionsbestimmung mit der Abtastvorrichtung dynamisch ausgeführt wird, während die Kugel gegen den zu analysierenden Teil ausgelenkt wird, wobei das dreidimensionale Positionierungssystem eingerichtet ist zum Bestimmen der augenblicklichen Position des Kugelmittelpunkts, sowie mit einem System der Oberflächenrekonstruktion aus einem deformierbaren Modell, welches zum Ausführen einer dem Kugelradius entsprechenden Korrektur eingerichtet ist.

8. System nach Anspruch 4, **dadurch gekennzeichnet, dass** ein Bild des angepassten Modells erstellt wird und dass dieses Bild angezeigt wird, indem seinen verschiedenen Bereichen Farben oder Dicken zugeordnet werden, welche die Dichte der in diesem Bereich unter Verwendung der Abtastvorrichtung gefundenen Punkte wiedergeben, wodurch der Genauigkeitsgrad des Modells des entsprechenden Bereiches angegeben wird.

9. System nach Anspruch 4 mit Mitteln, welche eingerichtet sind zum:
Bestimmen der Positionen, an welchen der Schienbeinknochen/Oberschenkelknochen zum Aufnehmen einer Schnittführung angebohrt werden muss, durch Berechnung; Anzeigen dieser Positionen auf dem angezeigten Bild des Schienbeinknochens/Oberschenkelknochens;
fortwährendes Anzeigen des Bildes einer Bohrführung, welche mit Lokalisierungsmitteln in dem dreidimensionalen Positionierungssystem ausgebildet ist, auf dem wiederhergestellten Bild des Schienbeinknochens/Oberschenkelknochens, wobei diese Führung Röhren umfasst, welche durch denselben Abstand getrennt sind wie Röhren der Schnittführung, die auf in dem Knochen befestigten Gewinden montiert werden soll, **dadurch gekennzeichnet, dass** die Bohrführung einen zentralen Punkt umfasst, der gegen den Knochen drücken kann und dessen Bild mit einem auf dem dargestellten Bild des Schienbeins/Oberschenkelknochens vorpositionierten Zielpunkt übereinstimmen muss.

10. System nach Anspruch 9, **dadurch gekennzeichnet, dass** die Bohrführung ferner Einstellmittel umfasst, welche betriebsfähig sind, sobald die Führung in ihre Position gebracht wurde.

11. System nach Anspruch 4, **dadurch gekennzeichnet, dass** die lokalisierbaren Elemente, welche mit dem Schienbein, dem Oberschenkelknochen und mit dem Sensor verknüpft sind, geometrisch unterschieden sind.

12. System nach Anspruch 11, **dadurch gekennzeichnet, dass** das mit dem Schienbein verknüpfte lokalisierbare Element die Form des Buchstabens T aufweist, das mit dem Oberschenkelknochen verknüpfte lokaliserbare Element die Form des Buchstabens F aufweist und dass mit dem Sensor verknüpfte lokaliserbare Element die Form des Buchstabens P aufweist.

13. System nach Anspruch 2, **dadurch gekennzeichnet, dass** die Position der Kniescheibenkomponente der Prothese erhalten wird durch Mittel, welche eingerichtet sind zum:
Bestimmen der Bewegungslinie eines Elementes, welches eine mit der externen Oberfläche der Kniescheibe verbundene lokaliserbare Position aufweist, in einer Kniebeugungsbewegung in einem dreidimensionalen Positionierungssystem;
Bestimmen des Punktes der internen Kniescheibenoberfläche, welcher besser mit dem Mittelpunkt der Einkerbung der Prothese des Oberschenkelknochens für einen ausgewählten Winkelbeugungsbereich übereinstimmt;
Führen der Platzierung eines Bohrgewindes zu diesem Punkt.
